# EUROPEAN PATENT SPECIFICATION

(11) **EP 1 722 836 B1**
(45) Date of publication and mention of the grant of the patent: **05.01.2011**
(21) Application number: 05711533.9
(22) Date of filing: 19.01.2005
(51) Int. Cl.: A61L 31/14

(54) **MESOPOROUS COATINGS FOR MEDICAL IMPLANTS COMPRISING CATALYST**
MESOPORÖSE BESCHICHTUNGEN FÜR MEDIZINISCHE IMPLANTATE MIT KATALYSATOR
REVETEMENTS ET DESSINS FONCTIONNELS POUR IMPLANTS MEDICAUX

(30) Priority: 20.01.2004 US 759605
(43) Date of publication of application: 22.11.2006
(73) Proprietor: Boston Scientific Limited, an Irish company, St. Michael, Barbados, West Indies (BB)
(72) Inventor: WEBER, Jan, Maple Grove, MN 55311 (US); HOLMAN, Tom, Princeton, MN 55371 (US); EIDENSCHINK, Tracee, Wayzata, MN 55391 (US); CHEN, John, Jianhua, Plymouth, MN 55446 (US)
(74) Representative: Joly, Jean-Jacques
(86) International application number: PCT/US2005/001437
(87) International publication number: WO 2005/072788

(56) References cited:
- EP-A- 1 424 095
- WO-A-03/070662
- DE-A1- 19 916 086
- DE-U1-202004 009 059
- GB-A- 2 362 892
- US-A1- 2002 150 684
- US-A1- 2003 135 261
- US-B1- 6 245 104

## Description

### Field of the Invention

The present invention regards medical implants for implantation within the body of a patient. More specifically, the present invention regards functional designs and functional mesoporous coating systems for implants that may be placed within the body of a patient.

### Background

Medical_implants may be natural, synthetic or hybrid materials that are intended to be placed within the body of a patient for prolonged periods of time. An implant may be used to, among other things, support collapsed vessels in the human vasculature, replace missing tissue or bone throughout the body of a patient, and supplement existing tissue, vessels, and structures. Implants may remain within the body of a patient for several days, weeks, and even years. Over time, the body's reaction to the implant can enhance the performance of the implant. For instance, when hard tissue, such as bone, is replaced with an implant, the body may, over time, absorb some or all of the implant and replace it with living tissue, a beneficial result in many instances. The body's reaction to an implant over time may also, however, be unwanted, reducing the implant's effectiveness. For instance, when vascular stents are placed within the vasculature of the body, restenosis of the surrounding vessel may occur in and around the stent as red blood cells and platelets attach themselves to the foreign implant. This renarrowing of the artery is counterproductive as it creates a medical condition much like the arteriosclerosis and hardening of the arteries that the stent was intended to cure. Further, in still other circumstances, rather than attacking the implant, the body may, instead, completely reject it, becoming inflamed or irritated after the implant's deployment and requiring the removal of the implant at some later time. This, too, is counterproductive to the effective and prolonged functioning of the implant.
U.S. Patent No. 6,245,104 describes a method of forming an iridium oxide coating on a metal stent. The iridium oxide coating can have catalytic properties. DE 19916086 describes a stent having a three concentric layers. Inner and outer surfaces of the stent are coated with a hard, brittle ceramic material, especially iridium oxide or titanium nitride. GB 2362892 describes an implant formed from a plastic substrate and an iridium-containing coating. The implant may be a vascular prosthesis.

### Summary of the Invention

The present invention regards an implant that may be uniquely shaped to enhance its functionality and that may also be coated with a coating system that affects its functionality. In one embodiment the implant may have a first surface that is covered with a filter material, the filter material being in contact with a catalyst that promotes the decomposition of hydrogen peroxide into hydrogen and oxygen. In this and other embodiments, this filter material may be made from ceramic materials and may be meso-porous. In another embodiment, the implant may or may not be coated with this system and may have at least one strut with a tapered cross-section, the cross-section becoming smaller in area when moving from a reference point on the Inside of the implant to the outside of the implant.

### Brief Description of the Drawings

Figure 1 is a side sectional view of a coated implant positioned within the vasculature of a patient in accord with the present invention.

Figure 2 is a side sectional view of another coated implant positioned within the vasculature of a patient in accord with the present invention.

Figure 3 is a side sectional view of another coated implant positioned within the vasculature of a patient.

Figure 4 Is a side sectional view of another coated implant.

Figure 5 is a side sectional view of the coated implant from Figure 4, after additional process steps have been taken, in accord with the present invention.

Figure 6 is a side sectional view of the coated implant from Figure 5, after additional process steps have been taken, also in accord with the present invention.

Figure 7 is a side view of an expandable stent.

Figure 8 is a cross-sectional view taken along line 8-8 of Figure 7.

Figure 9 is a side view of a spray coating process.

Figure 10 is a side sectional view of another coated implant.

Figure 11 is a side view of a coated expandable stent.

Figure 12 is a side sectional view of a coated implant .

### Detailed Description

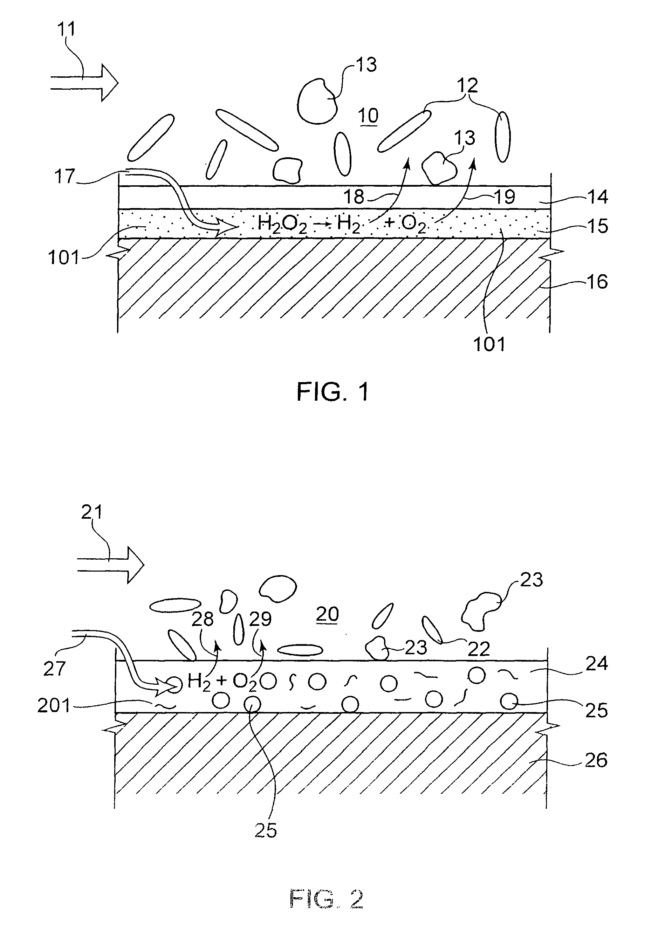

Figure 1 is a side sectional view of implant 16 after it has been placed in contact with a moving fluid 10, such as blood, within the body of a patient. The white blood cells 13 and red blood cells 12 of the fluid 10 are clearly labeled in Figure 1. Also visible in Figure 1 is the mesoporous layer 14, the catalytic layer 15 of the implant 16, and the therapeutic 101, which is within the catalytic layer 15. The direction of flow of fluid 10, which is blood in this embodiment but may be other fluids as well in this same embodiment and in others, is indicated with arrow 11.

In the present invention the coating 14 may function to prevent red blood cells 12 and white blood cells 13 from adhering to the implant 16 and from reaching the catalytic layer 15 of the implant 16. Thus, only particles and materials small enough to pass through the meso-porous layer 14 may reach the catalytic layer 15. Once there, the fluid may change under the influence of the catalyst, may come in contact with the therapeutic 101, and may then pass back out through the meso-porous layer to rejoin the flowing fluid 10. In Figure 1 the flow of the fluid 10 entering the catalytic layer 15 is shown with arrow 17 while the materials exiting the catalytic layer 15 are shown with arrows 18 and 19. Specifically, in Figure 1, hydroxide peroxide from the blood is shown entering the catalytic layer 15 where it will then react to create hydrogen and oxygen, which then exits the catalytic layer 15 as indicated by arrows 18 and 19. Also exiting the catalytic layer 15 would be therapeutic 101. While hydrogen peroxide is shown in Figure 1, other materials may also pass through the meso-porous layer 14 and may react with the catalyst before exiting back into the blood.

Depending upon which materials are filtered through the meso-porous layer, the catalyst may be chosen from numerous materials in order to promote the desired reaction. Likewise, various materials may also be used as a meso-porous layer 14 depending upon the use of the implant and the materials that will be filtered out. The materials that may comprise the mesoporous layer or coating include ceramic compositions, which generally improve the vascular compatibility of stents and other implants. Titanium, Zirconium, and Rutile Titanium Oxide, may also be used alone or with the addition of Hafnium to create the meso-porous layer. Other materials such as hydroxapatite, TiC, TaC, TiN, TaN, Ti, Cr, Al, Zr carbides, nitrides, oxides, oxycarbides, bucky paper and carbides may also be used to form the meso-porous layer. These materials may reduce restenosis of a vessel by decreasing the inflammatory response of the body to the implant and by retarding platelet adherence to the implant itself

The catalytic layer 15 may be made from porous or solid iridium oxide as well as from other catalysts such as manganese dioxide, platinum, and catalesen (potato enzyme), all of which decompose peroxide. In this embodiment, when the hydrogen peroxide contacts the Iridium Oxide, the peroxide reacts to become hydrogen and oxygen. In other embodiments, as mentioned above, other catalysts could be used to promote different reactions with different materials and fluids flowing through the meso-porous material.

In the present invention, it can be advantageous to increase the surface area of the catalyst and catalytic layer in order to provide a larger interface surface for the catalyst and to promote greater catalytic influence over the desired reaction. For instance, when iridium oxide is used as a catalyst, a porous IrO can provide a greater surface area than a solid IrO, and, consequently, a greater catalytic efficiency. Other materials and processes that increase the efficiency of the catalyst may be used as well.

A meso-porous material may generally have pores ranging from 2-50 nanometers. When pores are greater than 50 nanometers the ceramic is often considered macro-porous. Porous ceramics may be fabricated with a sol-gel process, using a polymer precursor that is later burned out to leave behind a porous ceramic structure. Since the pores in the ceramic are in the nanometer range the process relies on thermodynamics to drive the structure to an ordered state.

The meso-porous layer 14 in this embodiment may be made from numerous materials including titanium oxide, carbon nano-tubes, bucky paper, ceramics, and various other filter materials. These meso-porous materials may be created to contain uniformly sized holes or other passages that are patterned across the material, thereby turning the material into a sieve, allowing particles and materials smaller than the holes to pass but retarding the passage of particles that are larger than the holes. In some instances, the meso-porous material may be a filter whose pore size allows it to filter material as small as a single DNA chain from a fluid passing by or in contact with the meso-porous material.

With man-made meso-porous structures, the pore size can be adjusted from 0.3 nanometers to larger than 3000 nanometers. Typical meso-porous ceramics may be made from a sol-gel technique utilizing the block copolymer method to create a ceramic-polymer hybrid. The polymer can be removed thermally or chemically with a solvent to leave behind a porous ceramic structure. Another method of forming these structures involves using organic spheres with a specific diameter to form a colloid with a ceramic nanopowder. The powder fills in the gaps between the spheres when the colloid is evaporated. The spheres may then be dissolved thermally to create a porous structure. This process has been successful at forming 300 nanometer pores in titania, silica, and alumina with sample sizes of several millimeters. The shrinkage using this method has been found to be much lower than with a sol-gel process: 6 percent versus 30 percent.

Figure 2 is a side cross-sectional view of an implant 26 also in accord with the present invention. In Figure 2, the implant 26 may be partially or fully covered with a meso-porous layer 24 that contains or carries a catalyst 25 rather than covering one as depicted in Figure 1. In this embodiment, the fluid that was able to pass through the meso-porous layer would not have to pass all the way through the layer to reach a catalyst as in the earlier embodiment. Instead, the catalyst 25 is embedded throughout the layer 24 and may contact the fluid moving through the layer in order to promote the desired reactions. Once the desired reactions have occurred, the products of that reaction may then travel out of the layer 24 and may reenter the blood 20, which is moving in the direction of arrow 21. In so doing, the meso-porous layer may continue to prevent red blood cells 22 and white blood cells 23 from contacting the catalyst 25 and from adhering to the implant 26 while at the same time preventing the catalyst from contacting the smaller materials from the blood or other fluid. This sequence is identified by arrow 27 in Figure 2, which shows hydrogen peroxide entering the layer 24, reacting upon reaching the catalyst, and being released as hydrogen and oxygen in arrows 28 and 29.

Also shown in Figure 2 is a therapeutic 201 that is being carried by the implant 26. This therapeutic may be within the catalytic layer and may also be on areas that are not covered by catalyst. These non-covered areas could include, for example, the inside surfaces of a stent and the struts of the stent that are subject to great strain when the stent is expanded. Thus, the therapeutic may cover portions of the implant that are not covered by a meso-porous layer in addition to or in lieu of the areas of the implant that are themselves covered with a meso-porous layer.

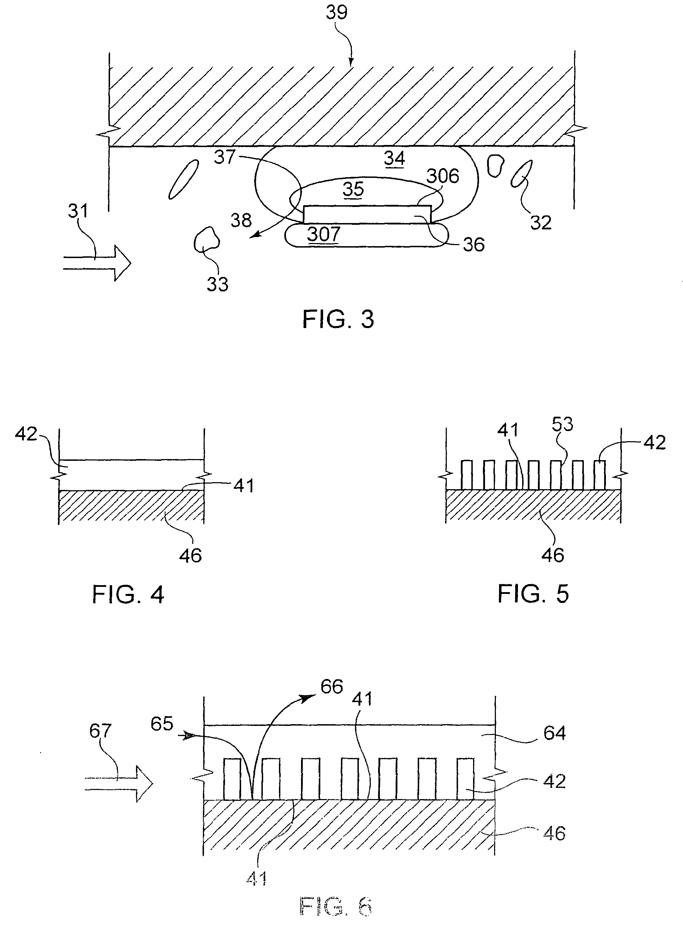

Figure 3 is a cross-section of a portion of an implant 36 shown within the vasculature of a patient. This implant 36 may be the strut of a stent or any other component of an implant. As can be seen, the illustrated portion of the implant 36 is partially covered by a catalyst 35 and a meso-porous structure 34 that is itself positioned against a vessel wall 39. As can also be seen in this embodiment the inwardly facing surface 306 of the implant 36 is not covered with a catalyst or a meso-porous structure but is, rather, covered with a therapeutic coating 307 that is exposed to the blood flowing past it.

In this embodiment, the meso-porous structure covers the catalyst 35. In so doing, the blood cells, platelets, and other materials in the blood, which are shown flowing in the direction of arrow 31, are less prone to adhere to the sides of the strut of the implant 36, where the blood would be more stagnant and somewhat disposed to clot. Arrows 37 and 38 of Figure 3 show the direction in which fluids may pass through the mesoporous structure 34, interface with the catalyst 35, and then pass back out into the blood. Alternatively, rather than having the therapeutic coating 307 on the fluid side of this strut as shown, the present invention may also include having the therapeutic carried by or adhered to the meso-porous layer or the catalyst as well.

Figures 4-6 show a sequential series of steps as may be employed to manufacture an implant consistent with the present invention. In Figure 6, which shows the final product, an implant 46 is covered with a catalyst 41, which is itself covered with a coating 42 that is itself coated with a meso-porous material. The coating 42 may be a block copolymer such as SIBS or some other polymer material. This polymer covering may contain a therapeutic or other material and may also be completely inert to the fluids that are intended to come in contact with it.

Figure 4 is illustrated as being the first step in the three depicted steps for manufacturing an implant in accord with the present invention. In Figure 4, an implant 46 is provided having a catalytic film 41 that is covered by a polymer coating 42. In Figure 5, the coating 42 has been removed in numerous spots creating access paths 53 to the catalyst 41. When the coating 42 is a polymer, the access paths 53 may be sub-micrometer in width as well as several micrometers in width. These access paths 53 may be cut into the coating 42 at random positions as well as in predetermined patterns. The paths 53 may be cut into the coating 42 by laser ablation using an excimer laser or some other type of laser. The excimer lasers used to perform the laser ablation may operate at 248 nm as well as wavelengths of 193 nm and shorter wavelengths. In some instances, a 248 nm ablation laser may also be used without affecting the implant surface. Conversely, when both the implant surface and the coating need to be etched or removed a femto-second laser may be used instead of or in addition to the excimer laser. This femto-second laser may allow both the coating and portions of the implant to be removed. Once the access paths 53 have been cut, a meso-porous layer 64 may be placed on top of the coating as shown in Figure 6.

As described throughout, this meso-porous layer may serve to filter certain materials and to retard the adherence of platelets and other materials to the implant. PI-b-PEO (poly[isoprene-b-ethylene oxide]), a block copolymer, may also be used with aluminiosilicate sol-gel precursors to fabricate the meso-porous ceramic in this and other embodiments. This material may self-assemble, due to thermodynamic forces, into ordered states based on the morphology of the copolymer. Once heat treatment is completed, the organics are burned off leaving behind a porous material. This sol-gel method allows for variation in the final material composition. Moreover, while a single block copolymer is used as a coating in this embodiment, the coating may be mixed with various other materials, for example, SIBS may be mixed with placitaxel, and Polyethermine may be mixed with Heparin.

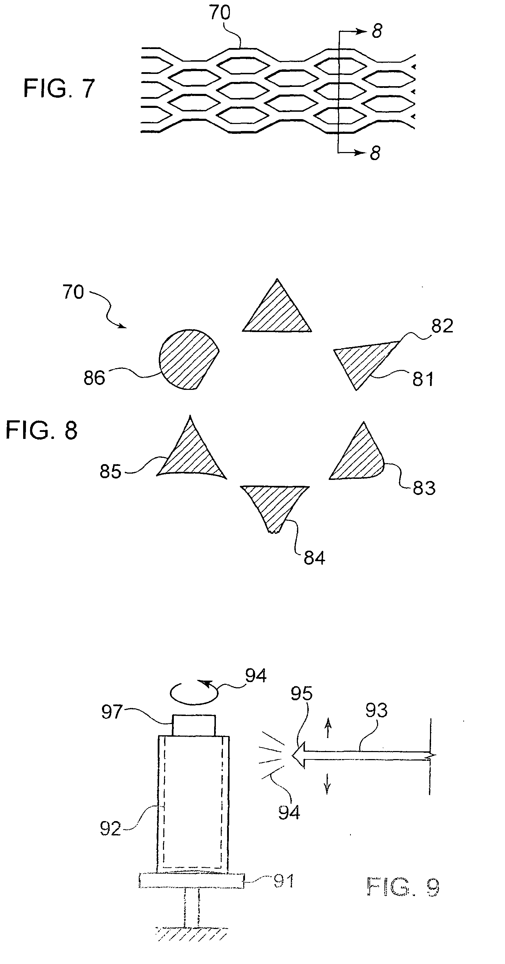

Figure 7 is a side view of an expandable coronary stent 70. Rather than use a catalyst to promote a decomposition of hydrogen peroxide or other material, this expandable stent has a modified cross section that promotes increased blood flow around the interface between the tissue and the stent walls, thereby taking advantage of catalysen in the blood.

Figure 8 is a sectional view along line 8-8 of Figure 7. As can be seen, the strut sections 81-86 of the stent 70 have tapered cross-sections with uniquely shaped distal ends 82. An advantage of having a strut section with a tapered cross-section is that the end of the strut that contacts the vessel to be enlarged is minimized. In so doing, the total surface area of the stent that is exposed to blood flowing through the vessel is increased. Moreover, by tapering the strut's profile, the strut's strength may be maintained while the contact area with the vessel may be reduced.

Some of the various strut cross-sections are shown in Figure 8. One or more of these cross-sections may be used on a single stent or other implant. If more than one cross-section is used, the cross-sections may alternate along a cross-sectional line, may be placed adjacent to one another along a cross-sectional line, may vary along the length of the stent, and may be used in other orientations in the stent as well. The cross-sections depicted in Figure 8 include the bull-nose cross-section 83, the undulating tip cross-section 84, the tapered cross-section 85, and the rounded cross-section 86. While five cross-sections are shown in Figure 8, it is more likely that a single stent or implant will have less than five different cross-sections. Nevertheless, five or even more than five different cross-sections of the stent strut are plausible. Still further, while no coating, therapeutic, and catalyst system is shown in Figure 8, the stent in Figure 8 may also be coated with the various systems described throughout this disclosure.

Figure 9 is a side view of a coating system. In this embodiment, an implant 92 may be placed on a platform 91 that may rotate as shown by arrow 94. Before, during, and after this rotation has begun and is occurring various materials may be sprayed from nozzle 95 onto the implant 92. The material 94 leaving the nozzle 95 as well as the supply line 93 to the nozzle 95 are labeled as such in Figure 9.

The material being sprayed from the nozzle may include the meso-porous materials and the catalytic materials described above. For example, when a two layer system, like the one pictured in Figure 1, is desired, the catalytic layer may be sprayed first, allowed to cure or dry and then the meso-porous layer may be sprayed on top of it. If carbon nano-tubes were being used to create the meso-porous layer, the nano-tubes may be dissolved in toluene, chloroform, Heparin or DNA and then sprayed on top of a previously applied catalyst. If the inside of the stent or other implant does not need to be coated, an insert 97 may be placed within the implant 92 to prevent spray 94 from entering the inside of the implant 92. Still further, if the system of Figure 2 were to be applied, rather than spraying two layers as described above, a single layer, containing the catalyst, therapeutic, and the meso-porous material may be sprayed concurrently.

In order to apply various types of coating systems, the supply line 93 may be connected to a network of storage vessels and valves that supply it with the appropriate material at the appropriate time during the coating process. Further, rather than using this spraying process for both layers of the system, a catalytic layer may be placed on top of an implant (e.g., using a plasma vapor deposition and electromechanical process to create a ceramic coating) and then subsequently covered with a nano-tube meso-porous layer by pouring a solution containing nano-tubes (e.g., THF w\nano-tubes) over the partially treated implant placed inside of a porous PVDF tube-filter. Once poured, the entire implant and tube filter may be spun at high speed to drive the solvents out, leaving behind a layer of bucky paper on top of the catalyst. The process may be repeated again if a second layer of bucky paper is desired.

Still further, the spraying process may be used to apply the catalytic layer while the mesoporous layer may be applied manually, when for instance, previously fabricated bucky paper is serving as the meso-porous layer.

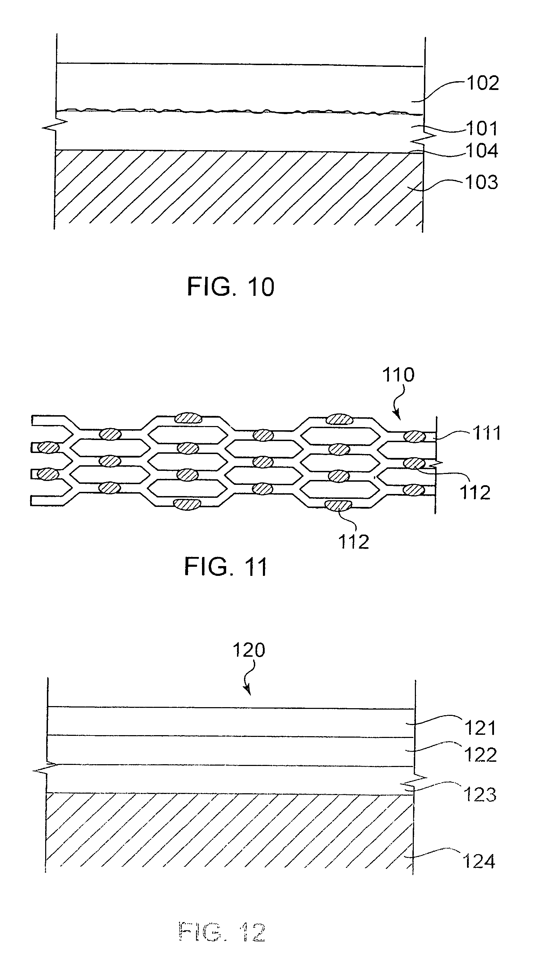

Figure 10 is a cross-section of an implant wall 103. In Figure 10, as well as in the others, the implant may be made with metalic and non-metalic materials. Likewise, the implant may be flexible, rigid or some variant of the two depending upon the desired application. In Figure 10 the implant is covered by TiO catalyst 104, IrO 101, and Bucky Paper 102.

The present invention may also include placing a layer of bucky paper on top of the layers 101 and 104. This bucky paper, which may consist of single wall and/or double wall carbon nano-tubes, may serve as the meso-porous layer described above.

Figure 11 is a side view of a stent implant also in accord with the present invention. In Figure 11 the meso-porous coating and the catalyst 112 have been placed at low strain areas of the stent 110 in order to reduce the risk of cracking the meso-porous layer when the stent is expanded within the vessel of a patient. As can be seen, the meso-porous coating and the catalyst 112 may be positioned on the stent 110 at uniform intervals along the stent. Alternatively, the meso-porous layer and the catalyst 112 may be positioned at non-uniform intervals along the stent 112 as well. The meso-porous layer and the catalyst 112 may be the mixed system described with Figure 2, the striated system described with Figure 1, as well as a blend of the two systems, where some stent struts are covered with striated systems and others are covered with mixed systems or some struts contain both systems.

Figure 12 is a cross-sectional side view of another implant . In Figure 12 the implant 124 contains a titanium coating 123, an IrO coating 122, and a bucky paper top layer 121. Here, the bucky paper 121 may function as the meso-porous layer while the IrO catalyst layer may be placed on a titanium layer rather than directly on the implant. Given the flexibility of bucky paper, this system 120 may be used on both rigid implants and expandable implants that may, otherwise, be non-compatible with more rigid ceramics. Moreover, in accord with the present invention these coatings and catalysts may be placed on the implant before or after the implant has been treated with a therapeutic. Still further, the nano-tubes may also be covered with a powder to promote reactions with fluids passing through them once they form the meso-porous structure. This powder may be platinum powder, manganese dioxide powder, and others.

The bucky paper described throughout this disclosure may be manufactured in accord with the following procedure. SWNTs may be commercially obtained as an aqueous suspension from Rice University (Houston, TX). The nanotube mats or bucky paper may be made by vacuum filtration through a poly(tetrafluoro ethylene) filter (Millipore LS, 47mm in diameter) of ~4g of a ~0.6 mg/ml nanotube suspension further diluted by the addition of ~80 ml of deionised water. The NT mat may be washed with 2x100 ml deionised water and 1x100ml methanol followed by drying at vacuum and 70°C/12 hours. In so doing, the typical nanotube mat may be between 15 and 35 µm thick and have a bulk density of 0.3 to 0.4 g/cm³, and a four point conductivity of 5000 S/cm. The nano-tubes (diameter 1.2-1.4 nm) may be synthesized by the laser vaporization method and purified by refluxing in nitric acid, washing and centrifugation followed by cross-flow filtration wherein the nano-tubes may spontaneously aggregate into bundles or "ropes" of ~10nm diameter and many microns in length. The nanotube mats may be peeled from the filter to produce free-standing films that may be used. In this example, measurement of actuation response was conducted using Seiko Instruments dynamic mechanical analyzer where a constant load was applied to the sample during immersion in the electrolyte and electrochemical cycling. Both triangular and square voltage waveforms were applied to the sample over various potential ranges. Both organic (0.1M tetrabutyl ammonium hexaflurophosphate in acetonitrile; TBAHFP in ACN) and aqueous (1M to 5M sodium chloride or 1 M hydrochloric acid) electrolytes were used.

The various therapeutics that may be applied to the above implants and their coatings may include pharmaceutically active compounds, nucleic acids with and without carrier vectors such as lipids, compacting agents (such as histones), viruses (such as adenovirus, andenoassociated virus, retrovirus, lentivirus and α-virus), polymers, hyaluronic acid, proteins, cells and the like, with or without targeting sequences. Specific examples of therapeutic agents used in conjunction with the present invention include, for example, pharmaceutically active compounds, proteins, cells, oligonucleotides, ribozymes, anti-sense oligonucleotides, DNA compacting agents, gene/vector systems (i.e., any vehicle that allows for the uptake and expression of nucleic acids), nucleic acids (including, for example, recombinant nucleic acids; naked DNA, cDNA, RNA; genomic DNA, cDNA or RNA in a non-infectious vector or in a viral vector and which further may have attached peptide targeting sequences; antisense nucleic acid (RNA or DNA); and DNA chimeras which include gene sequences and encoding for ferry proteins such as membrane translocating sequences ("MTS") and herpes simplex virus-1 ("VP22")), and viral, liposomes and cationic and anionic polymers and neutral polymers that are selected from a number of types depending on the desired application. Non-limiting examples of virus vectors or vectors derived from viral sources include adenoviral vectors, herpes simplex vectors, papilloma vectors, adeno-associated vectors, retroviral vectors, and the like. Non-limiting examples of biologically active solutes include anti-thrombogenic agents such as heparin, heparin derivatives, urokinase, and PPACK (dextrophenylalanine proline arginine chloromethylketone); antioxidants such as probucol and retinoic acid; angiogenic and anti-angiogenic agents and factors; anti-proliferative agents such as enoxaprin, angiopeptin, rapamycin, angiopeptin, monoclonal antibodies capable of blocking smooth muscle cell proliferation, hirudin, and acetylsalicylic acid; anti-inflammatory agents such as dexamethasone, prednisolone, corticosterone, budesonide, estrogen, sulfasalazine, acetyl salicylic acid, and mesalamine; calcium entry blockers such as verapamil, diltiazem and nifedipine; antineoplastic / antiproliferative / anti-mitotic agents such as paclitaxel, 5-fluorouracil, methotrexate, doxorubicin, daunorubicin, cyclosporine, cisplatin, vinblastine, vincristine, epothilones, endostatin, angiostatin and thymidine kinase inhibitors; antimicrobials such as triclosan, cephalosporins, aminoglycosides, and nitrofurantoin; anesthetic agents such as lidocaine, bupivacaine, and ropivacaine; nitric oxide (NO) donors such as linsidomine, molsidomine, L-arginine, NO-protein adducts, NO-carbohydrate adducts, polymeric or oligomeric NO adducts; anti-coagulants such as D-Phe-Pro-Arg chloromethyl ketone, an RGD peptide-containing compound, heparin, antithrombin compounds, platelet receptor antagonists, anti-thrombin antibodies, anti-platelet receptor antibodies, enoxaparin, hirudin, warfarin sodium, Dicumarol, aspirin, prostaglandin inhibitors, platelet inhibitors and tick antiplatelet factors; vascular cell growth promotors such as growth factors, growth factor receptor antagonists, transcriptional activators, and translational promotors; vascular cell growth inhibitors such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin; cholesterol-lowering agents; vasodilating agents; agents which interfere with endogenous vascoactive mechanisms; survival genes which protect against cell death, such as anti-apoptotic Bcl-2 family factors and Akt kinase; and combinations thereof. Cells can be of human origin (autologous or allogenic) or from an animal source (xenogeneic), genetically engineered if desired to deliver proteins of interest at the insertion site. Any modifications are routinely made by one skilled in the art. Polynucleotide sequences useful in practice of the invention include DNA or RNA sequences having a therapeutic effect after being taken up by a cell. Examples of therapeutic polynucleotides include anti-sense DNA and RNA; DNA coding for an anti-sense RNA; or DNA coding for tRNA or rRNA to replace defective or deficient endogenous molecules. The polynucleotides can also code for therapeutic proteins or polypeptides. A polypeptide is understood to be any translation product of a polynucleotide regardless of size, and whether glycosylated or not. Therapeutic proteins and polypeptides include as a primary example, those proteins or polypeptides that can compensate for defective or deficient species in an animal, or those that act through toxic effects to limit or remove harmful cells from the body. In addition, the polypeptides or proteins that can be injected, or whose DNA can be incorporated, include without limitation, angiogenic factors and other molecules competent to induce angiogenesis, including acidic and basic fibroblast growth factors, vascular endothelial growth factor, hif-1, epidermal growth factor, transforming growth factor α and β, platelet-derived endothelial growth factor, platelet-derived growth factor, tumor necrosis factor α, hepatocyte growth factor and insulin like growth factor; growth factors; cell cycle inhibitors including CDK inhibitors; anti-restenosis agents, including p15, p16, p18, p19, p21, p27, p53, p57, Rb, nFkB and E2F decoys, thymidine kinase ("TK") and combinations thereof and other agents useful for interfering with cell proliferation, including agents for treating malignancies; and combinations thereof. Still other useful factors, which can be provided as polypeptides or as DNA encoding these polypeptides, include monocyte chemoattractant protein ("MCP-1"), and the family of bone morphogenic proteins ("BMP's"). The known proteins include BMP-2, BMP-3, BMP-4, BMP-5, BMP-6 (Vgr-1), BMP-7 (OP-1), BMP-8, BMP-9, BMP-10, BMP-11, BMP-12, BMP-13, BMP-14, BMP-15, and BMP-16. Currently preferred BMP's are any of BMP-2, BMP-3, BMP-4, BMP-5, BMP-6 and BMP-7. These dimeric proteins can be provided as homodimers, heterodimers, or combinations thereof, alone or together with other molecules. Alternatively or, in addition, molecules capable of inducing an upstream or downstream effect of a BMP can be provided. Such molecules include any of the "hedgehog" proteins, or the DNA's encoding them

Polymers of the present invention may be hydrophilic or hydrophobic, and may be selected from the group consisting of polycarboxylic acids, cellulosic polymers, including cellulose acetate and cellulose nitrate, gelatin, polyvinylpyrrolidone, cross-linked polyvinylpyrrolidone, polyanhydrides including maleic anhydride polymers, polyamides, polyvinyl alcohols, copolymers of vinyl monomers such as EVA, polyvinyl ethers, polyvinyl aromatics, polyethylene oxides, glycosaminoglycans, polysaccharides, polyesters including polyethylene terephthalate, polyacrylamides, polyethers, polyether sulfone, polycarbonate, polyalkylenes including polypropylene, polyethylene and high molecular weight polyethylene, halogenated polyalkylenes including polytetrafluoroethylene, polyurethanes, polyorthoesters, proteins, polypeptides, silicones, siloxane polymers, polylactic acid, polyglycolic acid, polycaprolactone, polyhydroxybutyrate valerate and blends and copolymers thereof as well as other biodegradable, bioabsorbable and biostable polymers and copolymers. Coatings from polymer dispersions such as polyurethane dispersions (BAYHDROL®, etc.) and acrylic latex dispersions are also within the scope of the present invention. The polymer may be a protein polymer, fibrin, collagen and derivatives thereof, polysaccharides such as celluloses, starches, dextrans, alginates and derivatives of these polysaccharides, an extracellular matrix component, hyaluronic acid, or another biologic agent or a suitable mixture of any of these, for example.

In addition to the various teachings provided above, other examples of the present invention are also possible. For instance, the thicknesses of the various layers may be varied without straying from the teachings of this disclosure. In addition, poly-electrolyte technology may be used to allow multiple therapeutics to be released from a single implant with individual release rates. Still further, the entire implant may be made from a catalytic material that is then covered with a meso-porous material layer.

## Claims

1. A medical implant for deployment within a patient comprising:
an implant body having a first surface,
the first surface of the implant body covered with a filter material, the filter material covering or containing a catalyst that promotes the decomposition of hydrogen peroxide, and wherein the filter material is adapted to retard the passage of red blood cells and white blood cells.

2. The medical implant of claim 1 wherein the filter material is a mesoporous material.

3. The medical implant of claim 1 wherein the catalyst is positioned between the implant and the filter material.

4. The medical implant of claim 1 wherein the filter material covers the entire first surface of the implant.

5. The medical implant of claim 1 wherein the catalyst covers the entire first surface of the implant.

6. The medical implant of claim 1 wherein the filter material also comprises a therapeutic.

7. The medical implant of claim 1 wherein the catalyst also comprises a therapeutic.

8. The medical implant of claim 1 wherein the filter material covers the first surface and a second surface.

9. The medical implant of claim 1 wherein fluid in contact with the implant must pass through the filter material in order reach the catalyst.

10. The medical implant of claim 1 wherein the catalyst is titanium oxide.

11. The medical implant of claim 1 further comprising a polymer coating, the polymer coating positioned between the filter material and the catalyst.

12. The medical implant of claim 1 wherein the implant body includes a stent.

13. The medical implant of claim 12 wherein the filter material is positioned along a first face, a second face, and a third face of the stent.

14. The medical implant of claim 12 wherein the filter material does not cover at least a portion of the stent.

15. The medical implant of claim 1 further comprising a polymer layer covering the first surface of the implant body.

16. The medical implant of claim 15 wherein the catalyst is positioned between the polymer and the implant body.

17. The medical implant of claim 15 wherein portions of the polymer have been removed to create access paths through the polymer.

18. The medical implant of claim 17 wherein the implant body contains indentations coinciding with the location of at least one access path in the polymer.

19. The medical implant of claim 15 wherein the polymer comprises a therapeutic.

20. The medical implant of claim 1 wherein the filter material comprises carbon nanotubes.

21. The medical implant of claim 1 wherein the filter material comprises bucky paper.

22. The medical implant of claim 1 wherein the implant contains stent struts having tapered cross-sections, an inner surface of the strut having a larger area than an outer surface of the strut.

23. The medical implant of claim 1 wherein the first surface of the implant is covered by titanium, iridium oxide, and bucky paper.

24. The medical implant of claim 1 wherein regions of high strain of the implant when the implant is expanded are not covered with the filter material while regions of relatively lower strain when the implant is expanded are covered with the filter material.

25. The medical implant of claim 1 wherein the catalyst is chosen from a group consisting of manganese, iridium oxide, and platinum.

26. The medical implant of claim 1 wherein the catalyst has been previously treated to increase its surface area.

27. The medical implant of claim 1 wherein the filter material is bucky paper containing iridium oxide.

28. The medical implant of claim 1 wherein the implant body comprises a polymer.

29. The medical implant of claim 1 wherein the medical implant is a non-polymer and the catalyst promotes the decomposition of hydrogen peroxide to hydrogen and oxygen.

## Patentansprüche

1. Medizinisches Implantat zur Verwendung in einem Patienten umfassend:
einen Implantatkörper mit einer ersten Oberfläche,
wobei die erste Oberfläche des Implantatkörpers mit einem Filtermaterial bedeckt ist, wobei das Filtermaterial einen Katalysator, der die Zersetzung von Wasserstoffperoxyd begünstigt, bedeckt oder enthält und wobei das Filtermaterial geeignet ist, den Durchgang von roten Blutkörperchen und weißen Blutkörperchen zu verzögern.

2. Medizinisches Implantat nach Anspruch 1, wobei das Filtermaterial ein mesoporöses Material ist.

3. Medizinische Implantat nach Anspruch 1, wobei der Katalysator zwischen dem Implantat und dem Filtermaterial angeordnet ist.

4. Medizinisches Implantat nach Anspruch 1, wobei das Filtermaterial die gesamte erste Oberfläche des Implantats bedeckt.

5. Medizinisches Implantat nach Anspruch 1, wobei der Katalysator die gesamte erste Oberfläche des Implantats bedeckt.

6. Medizinisches Implantat nach Anspruch 1, wobei das Filtermaterial auch ein Therapeutikum umfaßt.

7. Medizinisches Implantat nach Anspruch 1, wobei der Katalysator auch ein Therapeutikum umfaßt.

8. Medizinisches Implantat nach Anspruch 1, wobei das Filtermaterial die erste Oberfläche und eine zweite Oberfläche bedeckt.

9. Medizinisches Implantat nach Anspruch 1, wobei eine Flüssigkeit, die in Kontakt mit dem Implantat steht, das Filtermaterial passieren muß, um den Katalysator zu erreichen.

10. Medizinisches Implantat nach Anspruch 1, wobei der Katalysator Titanoxyd ist.

11. Medizinisches Implantat nach Anspruch 1, ferner umfassend eine Polymerbeschichtung, wobei die Polymerbeschichtung zwischen dem Filtermaterial und dem Katalysator angeordnet ist.

12. Medizinisches Implantat nach Anspruch 1, wobei der Implantatkörper einen Stent umfaßt.

13. Medizinisches Implantat nach Anspruch 12, wobei das Filtermaterial auf einer ersten Seite, einer zweiten Seite und einer dritten Seite des Stents angeordnet ist.

14. Medizinisches Implantat nach Anspruch 12, wobei das Filtermaterial zumindest einen Abschnitt des Stents nicht bedeckt.

15. Medizinisches Implantat nach Anspruch 1, ferner umfassend eine Polymerschicht, die die erste Oberfläche des Implantatkörpers bedeckt.

16. Medizinisches Implantat nach Anspruch 15, wobei der Katalysator zwischen dem Polymer und dem Implantatkörper angeordnet ist.

17. Medizinisches Implantat nach Anspruch 15, wobei Abschnitte des Polymers entfernt worden sind, um Zugangswege durch das Polymer zu bilden.

18. Medizinisches Implantat nach Anspruch 17, wobei der Implantatkörper Einkerbungen umfaßt, die mit der Lage wenigstens eines Zugangsweges in dem Polymer übereinstimmen.

19. Medizinisches Implantat nach Anspruch 15, wobei das Polymer ein Therapeutikum umfaßt.

20. Medizinisches Implantat nach Anspruch 1, wobei das Filtermaterial Kohlenstoffnanoröhren umfaßt.

21. Medizinisches Implantat nach Anspruch 1, wobei das Filtermaterial Buckypapier umfaßt.

22. Medizinisches Implantat nach Anspruch 1, wobei das Implantat Stent-Streben mit konischen Querschnitten umfaßt, wobei eine innere Oberfläche der Strebe eine größere Fläche als eine äußere Oberfläche der Strebe besitzt.

23. Medizinisches Implantat nach Anspruch 1, wobei die erste Oberfläche des Implantats mit Titan, Iridiumoxyd und Buckypapier bedeckt ist.

24. Medizinisches Implantat nach Anspruch 1, wobei Abschnitte des Implantats, die bei Dehnung des Implantats hoher Belastung unterliegen, nicht mit dem Filtermaterial bedeckt sind während Abschnitte, die bei Dehnung des Implantats einer relativ niedrigen Belastung unterliegen, mit dem Filtermaterial bedeckt sind.

25. Medizinisches Implantat nach Anspruch 1, wobei der Katalysator aus der Gruppe, die aus Mangan, Iridiumoxyd und Platin besteht, ausgewählt ist.

26. Medizinisches Implantat nach Anspruch 1, wobei der Katalysator vorbehandelt wurde, um seine Oberfläche zu vergrößern.

27. Medizinisches Implantat nach Anspruch 1, wobei das Filtermaterial iridiumoxydenthaltendes Buckypapier ist.

28. Medizinisches Implantat nach Anspruch 1, wobei der Implantatkörper ein Polymer umfaßt.

29. Medizinisches Implantat nach Anspruch 1, wobei das medizinische Implantat ein Nichtpolymer ist und der Katalysator die Zersetzung von Wasserstoffperoxid zu Wasserstoff und Sauerstoff begünstigt.

## Revendications

1. Implant médical pour le déploiement chez un patient comprenant :
un corps d'implant ayant une première surface,
la première surface du corps d'implant couverte d'un matériau filtrant, le matériau filtrant couvrant ou contenant un catalyseur qui favorise la décomposition du peroxyde d'hydrogène, et où le matériau filtrant est adapté pour retarder le passage des globules rouges et des globules blancs.

2. Implant médical selon la revendication 1 où le matériau filtrant est un matériau mésoporeux.

3. Implant médical selon la revendication 1 où le catalyseur est positionné entre l'implant et le matériau filtrant.

4. Implant médical selon la revendication 1 où le matériau filtrant couvre toute la première surface de l'implant.

5. Implant médical selon la revendication 1 où le catalyseur couvre toute la première surface de l'implant.

6. Implant médical selon la revendication 1 où le matériau filtrant comprend aussi un agent thérapeutique.

7. Implant médical selon la revendication 1 où le catalyseur comprend aussi un agent thérapeutique.

8. Implant médical selon la revendication 1 où le matériau filtrant couvre la première surface et une seconde surface.

9. Implant médical selon la revendication 1 où un fluide en contact avec l'implant doit traverser le matériau filtrant pour atteindre le catalyseur.

10. Implant médical selon la revendication 1 où le catalyseur est l'oxyde de titane.

11. Implant médical selon la revendication 1 comprenant en outre un revêtement de polymère, le revêtement de polymère positionné entre le matériau filtrant et le catalyseur.

12. Implant médical selon la revendication 1 où le corps d'implant inclut un stent.

13. Implant médical selon la revendication 12 où le matériau filtrant est positionné le long d'une première face, d'une seconde face et d'une troisième face du stent.

14. Implant médical selon la revendication 12 où le matériau filtrant ne couvre pas au moins une partie du stent.

15. Implant médical selon la revendication 1 comprenant en outre une couche de polymère couvrant la première surface du corps d'implant.

16. Implant médical selon la revendication 15 où le catalyseur est positionné entre le polymère et le corps d'implant.

17. Implant médical selon la revendication 15 où des parties du polymère ont été retirées pour créer des voies d'accès à travers le polymère.

18. Implant médical selon la revendication 17 où le corps d'implant contient des échancrures coïncidant avec la position d'au moins une voie d'accès dans le polymère.

19. Implant médical selon la revendication 15 où le polymère comprend un agent thérapeutique.

20. Implant médical selon la revendication 1 où le matériau filtrant comprend des nanotubes de carbone.

21. Implant médical selon la revendication 1 où le matériau filtrant comprend une feuille de buckyballes.

22. Implant médical selon la revendication 1 où l'implant contient des éléments de compression de stent ayant les sections droites effilées, une surface interne de l'élément de compression ayant une plus grande aire qu'une surface externe de l'élément de compression.

23. Implant médical selon la revendication 1 où la première surface de l'implant est couverte par du titane, de l'oxyde d'iridium et une feuille de buckyballes.

24. Implant médical selon la revendication 1 où des régions de haute déformation de l'implant quand l'implant est expansé ne sont pas couvertes avec le matériau filtrant tandis que des régions de déformation relativement moindre quand l'implant est expansé sont couvertes avec le matériau filtrant.

25. Implant médical selon la revendication 1 où le catalyseur est choisi dans un groupe consistant en le manganèse, l'oxyde d'iridium et le platine.

26. Implant médical selon la revendication 1 où le catalyseur a été préalablement traité pour augmenter son aire.

27. Implant médical selon la revendication 1 où le matériau filtrant est une feuille de buckyballes contenant de l'oxyde d'iridium.

28. Implant médical selon la revendication 1 où le corps d'implant comprend un polymère.

29. Implant médical selon la revendication 1 où l'implant médical est un non-polymère et le catalyseur favorise la décomposition du peroxyde d'hydrogène en hydrogène et oxygène.
